(19) Europäisches Patentamt
European Patent Office
Office européen des brevets

(11) **EP 3 536 397 A1**

(12) **EUROPEAN PATENT APPLICATION**
published in accordance with Art. 153(4) EPC

(43) Date of publication:
**11.09.2019 Bulletin 2019/37**

(21) Application number: **17871086.9**

(22) Date of filing: **17.11.2017**

(51) Int Cl.:
**B01J 2/08** (2006.01)    **A61K 8/02** (2006.01)
**B01J 13/00** (2006.01)

(86) International application number:
**PCT/JP2017/041520**

(87) International publication number:
**WO 2018/092891 (24.05.2018 Gazette 2018/21)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **18.11.2016 JP 2016224760**

(71) Applicant: **Kao Corporation**
**Chuo-Ku**
**Tokyo 103-8210 (JP)**

(72) Inventors:
• **KITAGAWA Masafumi**
  **Wakayama-shi**
  **Wakayama 640-8580 (JP)**
• **HIRAMATSU Shinobu**
  **Wakayama-shi**
  **Wakayama 640-8580 (JP)**
• **FUKUDA Kimikazu**
  **Wakayama-shi**
  **Wakayama 640-8580 (JP)**

(74) Representative: **Hoffmann Eitle**
**Patent- und Rechtsanwälte PartmbB**
**Arabellastraße 30**
**81925 München (DE)**

(54) **METHOD FOR MANUFACTURING HYDROGEL PARTICLES**

(57)    A method for manufacturing hydrogel particles includes a step of cooling an aqueous solution, in which a gel agent forming a non-crosslinked hydrogel is dissolved, down to a temperature lower than the gel point of the aqueous solution. In the step of cooling, a cooled substance of the aqueous solution is stirred at least at the gel point, and after the gel point has been reached, a stirring energy of 400 kW·s/m$^3$ or higher is applied to the cooled substance of the aqueous solution with stirring at a required stirring power per unit volume of 0.8 kW/m$^3$ or higher.

EP 3 536 397 A1

**Description**

TECHNICAL FIELD

The present invention relates to a method for manufacturing hydrogel particles and a method for manufacturing a cosmetic product.

BACKGROUND ART

It has been known that hydrogel particles, in which dispersed particles including various types of functional materials are distributed, are contained in cosmetic products, drugs, and quasi drugs, for example.

Patent Document 1, for example, discloses a method for producing such hydrogel particles in which a dispersion, containing an oil component dispersed in an aqueous component solution of dissolved gel agent forming a non-crosslinked hydrogel, is prepared and sprayed into a gas phase to form droplets, which are then cooled and allowed to solidify.

Patent Document 2 discloses a method in which an O/W emulsion produced from an internal oil phase and an aqueous phase containing a hydrophilic polymer gel agent is dispersed in an external oil phase to prepare an O/W/O emulsion, and the O/W/O emulsion is cooled to solidify the aqueous phase thereof. Patent Document 3 discloses a method in which a water-containing liquid containing a high concentration of polysaccharide such as agar is heated, and then the resultant liquid is cooled with shear force being applied thereto to obtain a polysaccharide-containing composition having a low viscosity in a liquid state.

Furthermore, Patent Document 4 discloses a method for manufacturing a thickener, in which a hydrophilic compound having a gelling capability is dissolved in water or an aqueous component to prepare an aqueous solution, it is left to stand still to be cooled to form a gel, and then the gel is pulverized.

CITATION LIST

PATENT DOCUMENT

   PATENT DOCUMENT 1: Japanese Unexamined Patent Publication No. 2007-160277
   PATENT DOCUMENT 2: Japanese Unexamined Patent Publication No. 2001-97818
   PATENT DOCUMENT 3: Japanese Unexamined Patent Publication No. 2003-128588
   PATENT DOCUMENT 4: Japanese Unexamined Patent Publication No. 2001-342451

SUMMARY OF THE INVENTION

The present invention provides a method for manufacturing hydrogel particles, including a step of cooling an aqueous solution, in which a gel agent forming a non-crosslinked hydrogel is dissolved, down to a temperature lower than a gel point of the aqueous solution. In the step of cooling, a cooled substance of the aqueous solution is stirred at least at the gel point, and after the gel point has been reached, a stirring energy of 400 kW·s/m$^3$ or higher is applied to the cooled substance of the aqueous solution with stirring at a required stirring power per unit volume of 0.8 kW/m$^3$ or higher.

DESCRIPTION OF EMBODIMENTS

Embodiments will be described below in detail.

A method for manufacturing hydrogel particles according to an embodiment includes a step of cooling an aqueous solution in which a gel agent forming a non-crosslinked hydrogel is dissolved (hereinafter, also called "gel-agent aqueous solution") down to a temperature lower than the gel point of this gel-agent aqueous solution. In this step of cooling, a cooled substance of the gel-agent aqueous solution is stirred at least at the gel point, and after the gel point has been reached, a stirring energy of 400 kW·s/m$^3$ or higher is applied to the cooled substance of the gel-agent aqueous solution with stirring at a required stirring power per unit volume of 0.8 kW/m$^3$ or higher (hereinafter, also called "high-speed stirring").

In the method for manufacturing hydrogel particles according to the embodiment, in a process of cooling the gel-agent aqueous solution to the temperature lower than the gel point thereof, the cooled substance of the gel-agent aqueous solution is stirred at least at the gel point, and after the gel point has been reached, the stirring energy of 400 kW·s/m$^3$ or higher is applied to the cooled substance of the gel-agent aqueous solution with the high-speed stirring, whereby the non-crosslinked hydrogel obtained by gelation of the gel-agent aqueous solution can be obtained as a flowable gel in which fine particles aggregate. Thus, hydrogel particles can be manufactured by a simple method of stirring. Furthermore, by controlling this stirring energy, particle sizes of obtained hydrogel particles can be easily controlled.

Herein, the "hydrogel particle(s)" in the present application refers to one or more particles of non-crosslinked hydrogel. The "non-crosslinked hydrogel" refers to a gel made from a gel agent and water, which is a gel resulting from thermal reversibility of sol-gel as can be observed when the gel agent is agar, for example. The "gel agent" refers to a water-soluble organic compound, which is an agent for causing an aqueous solution in which this agent is dissolved to transition from sol to gel at its gel point (congealing point).

Examples of the gel agent that forms the non-crosslinked hydrogel include water-soluble polymers such as agar, carrageenan, gellan gum, xanthan gum, and high methoxyl pectin. As the gel agent, one, two, or more of these are suitably used. Among these, agar is more suitably used. The "agar" in the present application refers to hemicellulose containing galactan including 1,3-bond and 1,4-bond of galactose.

From the viewpoint of providing a good feel in use when the hydrogel particles thus obtained are used for a cosmetic product, for example, the jelly strength of the gel agent is suitably 19.6 kPa (200 g/cm$^2$) or higher and more suitably 50.0 kPa (510 g/cm$^2$) or higher. Furthermore, from the same viewpoint, the jelly strength is suitably 147 kPa (1500 g/cm$^2$) or lower and more suitably 127 kPa (1300 g/cm$^2$) or lower. The jelly strength of the gel agent is suitably in the range of 19.6 kPa to 147 kPa (200 g/cm$^2$ to 1500 g/cm$^2$) and more suitably in the range of 50.0 kPa to 127 kPa (510 g/cm$^2$ to 1300 g/cm$^2$). The jelly strength of the gel agent can be determined by the Nikkansui method. Specifically, the jelly strength of the gel agent can be determined by preparing a 1.5 mass% aqueous solution of the gel agent, leaving this aqueous solution to stand still for solidification at 20°C for 15 hours to form a non-crosslinked hydrogel, applying a load to this hydrogel with a Nikkansui-type jelly strength meter (manufactured by Kiya Corporation), and measuring the maximum mass (g) per surface area of 1 cm$^2$ when the non-crosslinked hydrogel can withstand the load at 20°C for 20 seconds.

From the viewpoint of manufacturing feasibility, the content of the gel agent in the gel-agent aqueous solution is suitably 0.10 mass% or higher, more suitably 0.30 mass% or higher, still more suitably 0.40 mass% or higher, and yet more suitably 0.50 mass% or higher. Furthermore, from the viewpoint of providing a good feel in use when the obtained hydrogel particles are used for a cosmetic product, for example, the content of the gel agent is suitably 8.0 mass% or lower, more suitably 7.0 mass% or lower, still more suitably 6.0 mass% or lower, yet more suitably 5.0 mass% or lower, and even still more suitably 3.0 mass% or lower. From all these viewpoints, the content of the gel agent in the gel-agent aqueous solution is suitably in the range of 0.10 mass% to 8.0 mass%, more suitably in the range of 0.30 mass% to 7.0 mass%, still more suitably in the range of 0.40 mass% to 6.0 mass%, yet more suitably in the range of 0.50 mass% to 5.0 mass%, and even still more suitably in the range of 0.50 mass% to 3.0 mass%.

From the viewpoint of solidification ability at room temperature, the gel point (congealing point) of the gel-agent aqueous solution is suitably 30°C or higher. From the viewpoint of its solubility during manufacturing, the gel point is suitably 50°C or lower and more suitably 45°C or lower. From all these viewpoints, the gel point of the gel-agent aqueous solution is suitably in the range of 30°C to 50°C and more suitably in the range of 30°C to 45°C. The gel point of the gel-agent aqueous solution can be determined by putting about 10 ml of the gel-agent aqueous solution into a medium-sized test tube (having a diameter of 1.5 cm × 16 cm), inserting a thermometer thereinto, letting it stand to cool, tilting the test tube, and reading the temperature when the surface of the solution has become immobilized.

The gel-agent aqueous solution may be a dispersion in which dispersed particles are distributed. Specifically, the obtained hydrogel particles may have a particle body of a non-crosslinked hydrogel in which dispersed particles are distributed. Examples of the dispersed particles include oil components, water-insoluble complexes containing catechins, and powder for cosmetic products.

Such an oil component is disclosed in detail in Patent Document 1. The dispersed particles of the oil component contain at least one of a solid fat or a liquid oil. Herein, the "solid fat" in the present application refers to an oil and fat having a melting point of 35°C or higher, and the "liquid oil" refers to an oil and fat having a melting point lower than 35°C.

Examples of the solid fat include solid ceramides, solid sphingolipids, solid paraffins, solid higher alcohols, vaselines, solid silicones, solid oils, and solid perfumes.

Examples of the solid ceramides include N-(2-hydroxy-3-hexadesiloxypropyl)-N-hydroxyethyl hexadecanamide. Examples of the solid sphingolipids include phytosphingosine. Examples of the solid paraffins include paraffin waxes and microcrystalline waxes listed in JIS K 2235 and ceresin. Examples of the solid higher alcohols include cetyl alcohol, stearyl alcohol, arachidyl alcohol, and behenyl alcohol. Examples of the solid silicones include alkyl-modified silicones and polymeric silicone alkyl co-modified acrylic resin. Examples of the solid oils include hardened oils and higher fatty acids. Examples of the hardened oils include hydrogenated oil, the feedstock oil of which is coconut oil, palm oil, or beef tallow. Examples of the higher fatty acids include palmitic acid, behenic acid, and stearic acid. Examples of the solid perfumes include menthol and cedrol.

From the viewpoint of achieving the effect of the solid fat, the content of the solid fat in the dispersed particles of the oil component is suitably 1.0 mass% or higher, more suitably 6.0 mass% or higher, still more suitably 10 mass% or higher, and yet more suitably 19 mass% or higher, and is also suitably 80 mass% or lower, more suitably 70 mass% or lower, and still more suitably 50 mass% or lower. The content of the solid fat in the dispersed particles of the oil component is suitably in the range of 1.0 mass% to 80 mass%, more suitably in the range of 6.0 mass% to 80 mass%, still more suitably in the range of 10 mass% to 70 mass%, and yet more suitably in the range of 19 mass% to 50 mass%.

Examples of the liquid oils include liquid skin-protecting agents, liquid oils, and liquid perfumes.

Examples of the liquid skin protecting agents include: liquid fats such as liquid paraffin, liquid ester oils such as methoxy octyl cinnamate, liquid higher alcohols, liquid squalane and liquid glycerides; liquid ceramides such as cetyloxypropylglyceryl methoxypropyl myristamide; and liquid sphingolipids such as 1-(2-hydroxyethylamino)-3-isostearyloxy-2-propanol. Examples of the liquid oils include: liquid hydrocarbon oils, liquid vegetable oils, liquid fatty acids; liquid oils and fats including liquid ethylene glycol di-fatty acid esters (the number of carbon atoms in the fatty acid is 12 or larger and 36 or smaller) and liquid dialkyl ethers (the number of carbon atoms is 12 or larger and 36 or smaller); and liquid silicones.

From the viewpoint of achieving the effect of the liquid oil, the content of the liquid oil in the dispersed particles of the oil component is suitably 10 mass% or higher, more suitably 20 mass% or higher, still more suitably 30 mass% or higher, and yet more suitably 50 mass% or higher. Furthermore, from the viewpoint of providing a good feel in use when the obtained hydrogel particles are used for a cosmetic product, for example, the content of the liquid oil is suitably 99 mass% or lower, more suitably 94 mass% or lower, still more suitably 90 mass% or lower, and yet more suitably 81 mass% or lower. From all these viewpoints, the content of the liquid oil in the dispersed particles of the oil component is suitably in the range of 55 mass% to 99 mass%, more suitably in the range of 20 mass% to 94 mass%, still more suitably in the range of 30 mass% to 90 mass%, and yet more suitably in the range of 50 mass% to 81 mass%.

As the solid fat and the liquid oil contained in the dispersed particles of the oil component, one, two, or more of these are suitably used.

The form of the dispersed particles of the oil component is not limited to a particular one, and may be a W/O emulsion, for example. Examples of substances that the dispersed particles of the oil component may contain other than the solid fat and the liquid oil include: inorganic ultraviolet absorbents such as titanium oxide and zinc oxide; organic ultraviolet absorbents such as bis-ethylhexyloxyphenol methoxyphenyl triazine, ethylhexyl triazone, diethylamino hydroxybenzoyl hexyl benzoate, dimethoxybenzylidene dioxoimidazolidine octyl propionate, and t-butyl methoxybenzoyl methane; and fat-soluble vitamins such as vitamin A, vitamin D, vitamin E, and vitamin K.

Water-insoluble complexes containing catechins are disclosed in detail in Japanese Unexamined Patent Publications Nos. 2010-131479 and 2011-136983. Dispersed particles of the water-insoluble complexes containing catechins include catechins and polymers that form the water-insoluble complexes with the catechins.

The catechins are non-polymer catechins, for example, and examples thereof include non-epicatechins such as catechin, gallocatechin, catechin gallate, and gallocatechin gallate; and epicatechins such as epicatechin, epigallocatechin, epicatechin gallate, and epigallocatechin gallate. As the catechins, one, two, or more of these are suitably used.

Examples of the polymers forming water-insoluble complexes with catechins include polyvinylpyrrolidone (PVP), polyvinyl alcohol (PVA), hydroxyethyl cellulose (HEC), methylcellulose (MC), hydroxypropyl cellulose (HPC), hydroxypropyl methylcellulose (HPMC), polyethylene glycol (PEG), polyglycerol fatty acid esters, polyoxyethylene alkyl ethers, polyoxyethylene sorbitan alkyl esters (polyoxyethylene sorbitan fatty acid esters), gelatin, and casein sodium. As the polymer, one, two, or more of these are suitably used. Polyvinylpyrrolidone (PVP), hydroxyethyl cellulose (HEC), methylcellulose (MC), hydroxypropyl cellulose (HPC), or polyethylene glycol (PEG) is more suitably used. Polyvinylpyrrolidone (PVP) is particularly suitably used.

Examples of the dispersed particles of powders for cosmetic products include: metal soap powders such as zinc stearate, aluminum stearate, calcium stearate, and zinc myristate; resin powders such as nylon powder, polymethyl methacrylate powder, acrylonitrile-methacrylic acid copolymer powder, vinylidene chloride-methacrylic acid copolymer powder, polystyrene powder, organopolysiloxane elastomer powder, and polymethylsilsesquioxane powder; inorganic powders such as titanium oxide, black titanium oxide, iron blue, ultramarine, red iron oxide, yellow iron oxide, black iron oxide, zinc oxide, aluminum oxide, silicon dioxide, magnesium oxide, zirconium oxide, magnesium carbonate, calcium carbonate, chromium oxide, chromium hydroxide, carbon black, aluminum silicate, magnesium silicate, magnesium aluminum silicate, mica, synthetic mica, synthetic sericite, sericite, talc, kaolin, silicon carbide, barium sulfate, bentonite, smectite, and boron nitride; photoluminescent powders such as bismuth oxychloride, mica titanium, iron oxide coated mica, iron oxide mica titanium, organic pigment treated mica titanium, and aluminum powder; organic powders such as wool powder, silk powder, and microcrystalline cellulose; dye powders such as silica, organic tar-based pigments, and organic dye lake pigments; and composite powders such as particulate titanium oxide-coated mica titanium, particulate zinc oxide-coated mica titanium, barium sulfate-coated mica titanium, titanium oxide-containing silicon dioxide, and zinc oxide-containing silicon dioxide. As the power for cosmetic products, one, two, or more of these are suitably used.

When the gel-agent aqueous solution is a dispersion, in the dispersion, one type of dispersed particles of the above-described examples may be distributed, or two or more types of dispersed particles thereof may be distributed.

From the viewpoint of achieving the effect of the dispersed particles, the content of the dispersed particles in the gel-agent aqueous solution that is a dispersion is suitably 1 mass% or higher, more suitably 2 mass% or higher, and still more suitably 3 mass% or higher. Furthermore, from the viewpoint of providing a good feel in use when the obtained hydrogel particles are used for a cosmetic product, for example, the content of the dispersed particles is suitably 60 mass% or lower, more suitably 55 mass% or lower, and still more suitably 50 mass% or lower. From all these viewpoints, the content of the dispersed particles is suitably in the range of 1 mass% to 60 mass%, more suitably in the range of 2 mass% to 55 mass%, and still more suitably in the range of 3 mass% to 50 mass%.

Examples of the particle shape of each dispersed particle include a spherical shape, a plate-like shape, and a needle-like shape.

From the viewpoint of safety, the volume-mean particle size of the dispersed particles is suitably 0.0100 $\mu$m or larger, more suitably 0.100 $\mu$m or larger, and still more suitably 1.00 $\mu$m or larger. Furthermore, from the viewpoint of providing a good feel in use when the obtained hydrogel particles are used for a cosmetic product, for example, and also from the viewpoint of manufacturing feasibility, the volume-mean particle size is suitably 100 $\mu$m or smaller, more suitably 50.0 $\mu$m or smaller, and still more suitably 20.0 $\mu$m or smaller. From all these viewpoints, the volume-mean particle size of the dispersed particles is suitably in the range of 0.0100 $\mu$m to 100 $\mu$m, more suitably in the range of 0.100 $\mu$m to 50.0 $\mu$m, and still more suitably in the range of 1.00 $\mu$m to 20.0 $\mu$m. The volume-mean particle size of the dispersed particles can be determined by measurement based on a laser diffraction/scattering method with a laser diffraction/scattering particle size distribution analyzer (e.g., LA-920 manufactured by HORIBA, Ltd.).

When the gel-agent aqueous solution is a dispersion, in the gel-agent aqueous solution that is a dispersion, at least one of a dispersant for distributing the dispersed particles or an emulsifier (hereinafter, also called "dispersant, etc.") is suitably contained.

Examples of the dispersant, etc. include polymer emulsifying and dispersing agents, anionic surfactants, cationic surfactants, nonionic surfactants, and amphoteric surfactants.

Examples of the polymer emulsifying and dispersing agents include a copolymer of acrylic acid and alkyl methacrylate, a complex in which an amphoteric polymer compound and a higher fatty acid are synthesized together as described in Japanese Unexamined Patent Publication No. H7-100356, water-soluble amphiphilic polymer electrolytes as respectively described in Japanese Unexamined Patent Publications Nos. H8-252447 and H9-141079, water-soluble crosslinked amphiphilic polymer electrolytes as respectively described in Japanese Unexamined Patent Publications Nos. H9-141080 and H9-141081, an acrylate-based copolymer as described in Japanese Unexamined Patent Publication No. H10-53625, polysaccharide derivatives as respectively described in Japanese Patent No. 3329689 and Japanese Unexamined Patent Publications Nos. H10-330401 and H11-106401, synthetic polymer compounds such as polyvinyl pyrrolidone, polyvinyl alcohol and their derivatives, polyacrylamides and ethylene oxide adducts of alkyl phenol formaldehyde condensates, and natural polymer compounds such as Guayagamu, karaya gum, tragacanth gum, gum arabic, arabinogalactan, and casein.

Examples of the anionic surfactants include sodium lauryl sulfate, sodium stearate, and sodium polyoxyethylene lauryl ether phosphate. Examples of the cationic surfactants include lauryl trimethyl ammonium chloride, stearyl amine acetate, and stearyl amine acid. Examples of the nonionic surfactants include sorbitan fatty acid esters, polyoxyethylene sorbitan fatty acid esters, and polyoxyethylene sorbitol fatty acid esters. Examples of the amphoteric surfactants include alkyldimethylamino acetic acid betaine and lecithin.

As the dispersant, etc. one type or two or more types of these are suitably used.

From the viewpoint of dispersion and emulsion stability, the content of the dispersant, etc. in the gel-agent aqueous solution that is a dispersion is suitably 0.01 mass% or higher, more suitably 0.05 mass% or higher, and still more suitably 0.1 mass% or higher. Furthermore, from the viewpoint of safety, the content of the dispersant, etc. is suitably 5 mass% or lower, more suitably 3 mass% or lower, and still more suitably 1 mass% or lower. From all these viewpoints, the content of the dispersant, etc. is suitably in the range of 0.01 mass% to 5 mass%, more suitably in the range of 0.05 mass% to 3 mass%, and still more suitably in the range of 0.1 mass% to 1 mass%.

The gel-agent aqueous solution may contain one, two, or more antiseptic agents selected from the group consisting of methyl parahydroxybenzoate, isopropyl methyl phenol, ethanol, phenoxyethanol, dehydroacetic acid and salts thereof, for example. The gel-agent aqueous solution may also contain water-soluble vitamins such as vitamin B and vitamin C. In addition to these, the gel-agent aqueous solution may further contain one, two, or more selected from the group consisting of moisturizers, antiperspirants, antimicrobial agents, and germicides.

In the step of cooling, from the viewpoint of dissolving the gel agent quickly, the temperature of the gel-agent aqueous solution before being cooled is suitably equal to or higher than the dissolution temperature of the gel agent and also equal to or lower than the boiling point of water. For example, when the gel agent is agar, the temperature of the gel-agent aqueous solution before being cooled is suitably 75°C or higher and more suitably 80°C or higher, and is also suitably 100°C or lower. When the gel agent is agar, the temperature of the gel-agent aqueous solution before being cooled is suitably in the range of 75°C to 100°C and more suitably in the range of 80°C to 100°C. Herein, to promote dissolution of the gel agent, the temperature of the gel-agent aqueous solution may be raised to 100°C or higher under increased pressure.

From the viewpoint of increasing the productivity, the average cooling speed in the step of cooling is suitably 0.1°C/min or higher, more suitably 0.5°C/min or higher, and still more suitably 0.8°C/min or higher. From the viewpoint of obtaining fine hydrogel particles, the average cooling speed is suitably 5°C/min or lower, more suitably 4°C/min or lower, and still more suitably 3°C/min or lower. The average cooling speed in the step of cooling is suitably in the range of 0.1°C/min to 5°C/min, more suitably in the range of 0.5°C/min to 4°C/min, and still more suitably in the range of 0.8°C/min to 3°C/min. This average cooling speed is a value obtained when the temperature difference between the cooling-start temperature and the temperature at the time when high-speed stirring described later has just been completed is divided by an elapsed time from the cooling-start time to this high-speed stirring completion time. The cooling speed after the gel point has been reached may be the same as, or may be higher than, or may be lower than the cooling speed before the gel point is reached. In this cooling process, a period of time when the cooling speed is 0 and a predetermined temperature (e.g., the gel point) is maintained may be set.

In the method for manufacturing hydrogel particles according to the embodiment, in the step of cooling, the cooled substance of the gel-agent aqueous solution is stirred at least at the gel point. At this time, after the gel point has been reached, a stirring energy of 400 kW·s/m³ or higher is applied to the cooled substance of the gel-agent aqueous solution by high-speed stirring. Examples of a stirrer used for this high-speed stirring include a disperser, a homogenizer, a grinder, a cutter mill, a ball mill, and a jet mill.

This high-speed stirring is suitably performed also for the gel-agent aqueous solution before the gel point is reached. Thus, the high-speed stirring of the gel-agent aqueous solution or the cooled substance thereof is suitably performed continuously over a period from before the temperature of the gel-agent aqueous solution or the cooled substance thereof reaches the gel point to after the gel point has been reached.

This high-speed stirring is suitably performed while the temperature of the cooled substance of the gel-agent aqueous solution is maintained at a certain temperature or within a certain temperature range equal to or lower than the gel point. In this case, the high-speed stirring may be performed in a process of cooling to lower the temperature of the cooled substance of the gel-agent aqueous solution from the gel point to the certain temperature or to within the certain temperature range.

From the viewpoint of stability of the obtained hydrogel particles, the temperature difference between the temperature of the cooled substance of the gel-agent aqueous solution during high-speed stirring and the gel point is suitably 0°C or larger, more suitably 1.0°C or larger, and still more suitably 1.5°C or larger. Furthermore, from the viewpoint of operability, the temperature difference is suitably smaller than 20.0°C, more suitably 18.0°C or smaller, and still more suitably 12.0°C or smaller. The temperature difference between the temperature of the cooled substance of the gel-agent aqueous solution during high-speed stirring and the gel point is suitably equal to or larger than 0°C and smaller than 20.0°C, more suitably in the range of 1.0°C to 18.0°C, and still more suitably in the range of 1.5°C to 12.0°C. From the viewpoint of increasing the yield and the controllability of particle sizes of the obtained hydrogel particles, the temperature difference between the temperature of the cooled substance of the gel-agent aqueous solution during high-speed stirring and the gel point is yet more suitably 7.0°C or smaller and even still more suitably 3.0°C or smaller.

At the gel point, gelation of the gel-agent aqueous solution starts. Whereas, at the gel point or in a temperature range lower than the gel point by several degrees, in the cooled substance of the gel-agent aqueous solution, a gel-agent aqueous solution and a non-crosslinked hydrogel obtained by gelation of this solution coexist. It is thought that, when this cooled substance of the gel-agent aqueous solution is stirred at high speed at the gel point or in the temperature range lower than the gel point by several degrees, gelation of the gel-agent aqueous solution is suppressed, whereby formation of an aggregated gel is suppressed, and also substances obtained by gelation of the gel-agent aqueous solution are kept from sticking to an inner wall, for example, of a stirring tank and being scattered and lost, and consequently the yield of the hydrogel particles can be increased. It is also thought that, because the distributed state of nuclei forming a gel of the gel-agent aqueous solution is changed by high-speed stirring, there is a strong correlation between the stirring energy of the high-speed stirring and the particle size of the hydrogel particles, and consequently the controllability of particle sizes of the hydrogel particles can be increased.

The required stirring power per unit volume when the cooled substance of the gel-agent aqueous solution is stirred at high speed after the gel point has been reached is 0.8 kW/m³ or higher. From the viewpoint of obtaining fine hydrogel particles, the required stirring power per unit volume is suitably 1.0 kW/m³ or higher, more suitably 1.1 kW/m³ or higher, and still more suitably 1.2 kW/m³ or higher. Furthermore, from the viewpoint of reduction of load on the stirrer and operability thereof, the required stirring power per unit volume is suitably 15 kW/m³ or lower, more suitably 10 kW/m³ or lower, and still more suitably 7.0 kW/m³ or lower. This required stirring power per unit volume is suitably in the range of 1.0 kW/m³ to 15 kW/m³, more suitably in the range of 1.1 kW/m³ to 10 kW/m³, and still more suitably in the range of 1.2 kW/m³ to 7.0 kW/m³.

The stirring energy to be applied to the cooled substance of the gel-agent aqueous solution by high-speed stirring after the gel point has been reached is 400 kW·s/m³ or higher. From the viewpoint of obtaining fine hydrogel particles, the stirring energy is suitably 600 kW·s/m³ or higher, more suitably 800 kW·s/m³ or higher, still more suitably 1000 kW·s/m³ or higher, yet more suitably 1500 kW·s/m³ or higher, and even still more suitably 2000 kW·s/m³ or higher. From the viewpoint of increasing efficiency, the stirring energy is suitably 15000 kW·s/m³ or lower, more suitably 12000 kW·s/m³ or lower, still more suitably 10000 kW·s/m³ or lower, yet more suitably 8000 kW·s/m³ or lower, even still more suitably 6000 kW·s/m³ or lower, and most suitably 4000 kW·s/m³ or lower. This stirring energy is suitably in the range of 600 kW·s/m³ to 10000 kW·s/m³, more suitably in the range of 800 kW·s/m³ to 8000 kW·s/m³, still more suitably in the range of 1000 kW·s/m³ to 6000 kW·s/m³, and yet more suitably in the range of 1500 kW·s/m³ to 4000 kW·s/m³.

From the viewpoint of obtaining a particle size suitable to provide a good feel when being used for cosmetic products, for example, this stirring energy to be applied to the cooled substance of the gel-agent aqueous solution by high-speed stirring after the gel point has been reached is suitably 4000 kW·s/m³ or higher, more suitably 5000 kW·s/m³ or higher, still more suitably 6000 kW·s/m³ or higher, and yet more suitably 7000 kW·s/m³ or higher, and is also suitably 13000 kW·s/m³ or lower, more suitably 11000 kW·s/m³ or lower, still more suitably 10000 kW·s/m³ or lower, and yet more suitably 9000 kW·s/m³ or lower. This stirring energy is suitably in the range of 4000 kW·s/m³ to 13000 kW·s/m³, more suitably in the range of 5000 kW·s/m³ to 11000 kW·s/m³, still more suitably in the range of 6000 kW·s/m³ to 10000 kW·s/m³, and yet more suitably in the range of 7000 kW·s/m³ to 9000 kW·s/m³.

The "stirring energy" in the present application is determined based on the specific equation described in Japanese Unexamined Patent Publication No. 2007-161683. For example, when a homomixer or a disperser is used, the stirring energy is calculated by the following Equation (I).

$$\text{Stirring Energy (kW·s/m}^3)$$

$$= [\text{Required Stirring Power P (kW)}] / [\text{Process Liquid's Volume V (m}^3)] \times \text{Stirring Time (s)}$$

$$\text{(I)}$$

In this Equation (I), the required stirring power P (kW) can be measured by a publicly known method using a dynamometer, for example. Alternatively, the required stirring power can be calculated by the following Experimental Equation 1.

$$\text{Required Stirring Power P (kW)} = Np \times n^3 \times d^5 \times \rho/1000 \quad \text{(Experimental}$$

$$\text{Equation 1)}$$

where Np is power number (e.g., Np=1.5 in a homomixer having a stirring tank capacity smaller than 10 L, and Np=1.3 in a homomixer having a stirring tank capacity of 10 L or larger. Np=0.8 in a disperser.);
n is the number of revolutions for stirring (-/s);
d is the diameter of the impeller (m); and
$\rho$ is the density (kg/m³) of the process liquid.

If the high-speed stirring is performed also on the gel-agent aqueous solution before the gel point is reached, the required stirring power per unit volume may be the same as, or may be lower than, or may be higher than the required stirring power per unit volume after the gel point has been reached. The stirring energy to be applied to the gel-agent aqueous solution before the gel point is reached may be the same as, or may be lower than, or may be higher than the stirring energy to be applied to the cooled substance of the gel-agent aqueous solution after the gel point has been reached.

It is suitable that the high-speed stirring be changed to stirring at a required stirring power per unit volume lower than 0.8 kW/m³ (hereinafter, also called "low-speed stirring") to be completed or be stopped to be completed. After the high-speed stirring is completed, the cooled substance of the gel-agent aqueous solution may be stirred at low speed for aging or be left to stand still for aging. This aging is suitably performed while the temperature of the cooled substance of the gel-agent aqueous solution is being maintained at a certain temperature or within a certain temperature range equal to or lower than the gel point.

From the viewpoint of stability of the obtained hydrogel particles, the temperature difference between the aging temperature for the aging and the gel point is suitably 0°C or larger, more suitably 1.0°C or larger, and still more suitably 1.5°C or larger. Furthermore, from the same viewpoint, the temperature difference is suitably smaller than 20.0°C, more suitably 18.0°C or smaller, still more suitably 12.0°C or smaller, yet more suitably 7.0°C or smaller, and even still more suitably 3.0°C or smaller. From the viewpoint of stability of the obtained hydrogel particles, the temperature difference between the aging temperature for the aging and the temperature of the cooled substance of the gel-agent aqueous solution during high-speed stirring is suitably 10.0°C or smaller, more suitably 5.0°C or smaller, and still more suitably 3.0°C or smaller. The aging temperature may be the same as the temperature of the cooled substance of the gel-agent aqueous solution at the high-speed stirring completion time.

From the viewpoint of stability of the hydrogel particles, the aging time for the aging is suitably 10 minutes or longer, more suitably 30 minutes or longer, and still more suitably 60 minutes or longer. Furthermore, from the viewpoint of productivity, the aging time is suitably 300 minutes or shorter, more suitably 180 minutes or shorter, and still more suitably 120 minutes or shorter. After the high-speed stirring is completed, or after the aging is completed, the cooled substance of the gel-agent aqueous solution may be further cooled.

From the viewpoint of stability of the obtained hydrogel particles, the temperature of the hydrogel particles obtained in this step of cooling is suitably equal to or lower than the gel point, more suitably a temperature lower than the gel point by 1.0°C or more, and still more suitably a temperature lower than the gel point by 1.5°C or more. When the gel agent is agar, from the viewpoint of solidification ability at room temperature, the temperature of the hydrogel particles obtained in the step of cooling is suitably 5.0°C or higher, more suitably 8.0°C or higher, and still more suitably 10.0°C or higher. Furthermore, from the same viewpoint, this temperature is suitably 35.5°C or lower, more suitably 34.5°C or lower, and still more suitably 34.0°C or lower. When the gel agent is agar, the temperature of the hydrogel particles obtained in the step of cooling is suitably in the range of 5.0°C to 35.5°C, more suitably in the range of 8.0°C to 34.5°C, and still more suitably in the range of 10.0°C to 34.0°C.

From the viewpoint of providing a good feel in use when the obtained hydrogel particles are used for a cosmetic product, for example, the volume-mean particle size of the hydrogel particles obtained in the step of cooling is suitably 0.100 $\mu$m or larger, more suitably 10.0 $\mu$m or larger, and still more suitably 20.0 $\mu$m or larger, and is also suitably 10000 $\mu$m or smaller, more suitably 1000 $\mu$m or smaller, and still more suitably 600 $\mu$m or smaller. The volume-mean particle size of the hydrogel particles is suitably in the range of 0.100 $\mu$m to 10000 $\mu$m, more suitably in the range of 10.0 $\mu$m to 1000 $\mu$m, and still more suitably in the range of 20.0 $\mu$m to 600 $\mu$m. The volume-mean particle size of the hydrogel particles is determined by measurement based on a laser diffraction/scattering method using a laser diffraction/scattering particle size distribution analyzer (e.g., LA-920 manufactured by HORIBA, Ltd.) or a screening method. The laser diffraction/scattering method is suitably used for measurement of particles having a particle size of 1000 $\mu$m or smaller, and the screening method is suitably used for measurement of particles having a particle size exceeding 1000 $\mu$m.

The yield of the hydrogel particles obtained in the step of cooling is suitably 70 mass% or higher, more suitably 80 mass% or higher, and still more suitably 90 mass% or higher. This yield of the hydrogel particles is determined as a percentage of the mass of the collected hydrogel particles with respect to the mass of the prepared gel-agent aqueous solution.

In the method for manufacturing hydrogel particles according to the embodiment, in the step of cooling, it is suitable that the gel-agent aqueous solution and the cooled substance thereof be cooled in a single stirring tank, and also the cooled substance of the gel-agent aqueous solution be stirred at high speed therein. This stirring tank is suitably provided with a cooling mechanism such as a heat exchanger. Note that the gel-agent aqueous solution and the cooled substance may also be cooled or stirred at high speed while tanks for storing these solutions are changed.

The obtained hydrogel particles can, without being processed, be blended with a cosmetic component into a liquid phase to manufacture a cosmetic product.

In this case, the cosmetic component may be put into a stirring tank in which the hydrogel particles have been manufactured, or the obtained hydrogel particles and the cosmetic component may be put into a stirring tank different from the stirring tank in which the hydrogel particles have been manufactured. From the viewpoint of manufacturing hydrogel particles having a suitable particle size, it is suitable that the obtained hydrogel particles and the cosmetic component be put into a stirring tank different from the stirring tank in which the hydrogel particles have been manufactured.

Examples of the cosmetic component include: polyether-modified silicones; cosmetic oils; humectants; intercellular lipids such as ceramides; ultraviolet absorbents such as titanium oxide, zinc oxide, and extracts of animals and plants; vitamins such as fat-soluble vitamins and water-soluble vitamins; chelating agents; pH adjusting agents; antiseptic agents; dyes; perfumes; medicinal components such as whitening, analgesics, antipruritic agents, disinfectants, astringents, emollients, and hormonal agents; emulsifying agents; cleaning agents; and antioxidants. As the cosmetic component, one, two, or more of these are suitably used.

Examples of a liquid that forms the liquid phase include water, liquid oils such as silicone oil, and organic solvents. As the liquid, one, two, or more of these are suitably used. From the viewpoint of providing a good feel in use when the obtained hydrogel particles are used for the cosmetic product, for example, the liquid phase is suitably water, an aqueous solution, or an aqueous phase of a water dispersion.

When a cosmetic product, for example, is manufactured, the obtained hydrogel particles may be put into a liquid phase and also be crushed by a crushing method to obtain finer particles. Examples of a liquid that forms the liquid phase include water, liquid oils such as silicone oil, and organic solvents. As the liquid, one, two, or more of these are suitably used. From the viewpoint of providing a good feel in use when the obtained hydrogel particles are used for the cosmetic product, for example, the liquid phase is suitably water, an aqueous solution, or an aqueous phase of a water dispersion.

Examples of the crushing method include a method of passing a liquid phase containing the hydrogel particles through a sieve such as a wire mesh to crush the particles and a method of shearing a liquid phase containing the hydrogel particles with a dispersing machine such as a line mixer, a disperser, a homogenizer, a milder, and a homomixer to crush the particles.

From the viewpoint of crushing the hydrogel particles inexpensively and simply, among these methods, the method of passing a liquid phase containing the hydrogel particles through a sieve is suitable. Specifically, with an apparatus including a pipe and a sieve provided halfway through the pipe, a liquid phase containing the hydrogel particles may be caused to flow through the pipe, and the liquid phase containing the hydrogel particles may be passed through the sieve to further crush the hydrogel particles.

In the case of crushing the hydrogel particles with a sieve, the aperture of the sieve is appropriately selected based on the particle size of the hydrogel particles. From the viewpoint of providing a good feel in use when the obtained hydrogel particles are used for a cosmetic product, for example, the aperture of the sieve is suitably 1 $\mu$m or larger, more suitably 10 $\mu$m or larger, and still more suitably 50 $\mu$m or larger, and is also suitably 1000 $\mu$m or smaller, more suitably 800 $\mu$m or smaller, still more suitably 600 $\mu$m or smaller, yet more suitably 500 $\mu$m or smaller, and even still more suitably 400 $\mu$m or smaller. The aperture of the sieve is suitably in the range of 1 $\mu$m to 1000 $\mu$m, more suitably in the range of 10 $\mu$m to 800 $\mu$m, still more suitably in the range of 50 $\mu$m to 600 $\mu$m, yet more suitably in the range of 50 $\mu$m to 500 $\mu$m, and even still more suitably in the range of 50 $\mu$m to 400 $\mu$m.

From the viewpoint of reducing variations in particle size of the hydrogel particles, the liquid phase containing the hydrogel particles is suitably passed through the sieve a plurality of times. In this case, the liquid phase containing the hydrogel particles may be circulated to be passed through a single sieve or a plurality of identical sieves a plurality of times. Alternatively, the liquid phase containing the hydrogel particles may be passed through a plurality of sieves that are arranged in series either continuously or at intervals.

When the liquid phase containing the hydrogel particles is passed through a plurality of sieves, from the viewpoint of providing a good feel in use when the obtained hydrogel particles are used for a cosmetic product, for example, the number of the sieves is suitably two or greater and more suitably three or greater. Furthermore, from the viewpoint of productivity and cost efficiency, the number of the sieves is suitably ten or less and more suitably five or less. From all these viewpoints, the number of the sieves is suitably in the range of two to ten and more suitably in the range of three to five. The sieves may have either the same aperture or mutually different apertures. Alternatively, some of the sieves may have the same aperture and the other sieves may have different apertures.

When the sieves include sieves having mutually different apertures, the sieves are suitably arranged in the descending order of their apertures such that one of the sieves having the largest aperture is arranged most upstream of any other one of the sieves. In this case, in each adjacent pair of sieves having mutually different apertures among the sieves arranged in the descending order of their apertures such that the sieve having the largest aperture is arranged most upstream, the ratio of the aperture of the downstream sieve of the pair to the aperture of the upstream sieve thereof is suitably 1% or higher, more suitably 10% or higher, and still more suitably 20% or higher, and is also suitably 100% or lower, more suitably 90% or lower, and still more suitably 80% or lower from the viewpoint of manufacturing feasibility. In each adjacent pair of sieves having mutually different apertures among the sieves arranged in the descending order of their apertures such that the sieve having the largest aperture is arranged most upstream, the ratio of the aperture of the downstream sieve of the pair to the aperture of the upstream sieve thereof is suitably in the range of 1% to 100%, more suitably in the range of 10% to 90%, and still more suitably in the range of 20% to 80%. Furthermore, among the sieves arranged in the descending order of their apertures such that the sieve having the largest aperture is arranged most upstream, the ratio of the aperture of the most downstream sieve to the aperture of the most upstream sieve is suitably 0.1% or higher, more suitably 1% or higher, and still more suitably 5% or higher, and is also suitably 100% or lower, more suitably 90% or lower, and still more suitably 80% or lower from the viewpoint of manufacturing feasibility. Among the sieves arranged in the descending order of their apertures such that the sieve having the largest aperture is arranged most upstream, the ratio of the aperture of the most downstream sieve to the aperture of the most upstream sieve is suitably in the range of 0.1% to 100%, more suitably in the range of 1% to 90%, and still more suitably in the range of 5% to 80%.

Into the liquid phase, a cosmetic component may be put in advance together with the hydrogel particles before crushing. When the cosmetic component is added to the liquid phase in advance in this manner, a cosmetic product can be manufactured while the hydrogel particles are crushed into further finer particles. Examples of the cosmetic component include: polyether-modified silicones; cosmetic oils; humectants; intercellular lipids such as ceramides; ultraviolet absorbents such as titanium oxide, zinc oxide, and extracts of animals and plants; vitamins such as fat-soluble vitamins and water-soluble vitamins; chelating agents; pH adjusting agents; antiseptic agents; dyes; perfumes; medicinal components such as whitening, analgesics, antipruritic agents, disinfectants, astringents, emollients, and hormonal agents; emulsifying agents; cleaning agents; and antioxidants. As the cosmetic component, one, two, or more of these are suitably used.

Regarding the embodiment described above, the following configurations are further disclosed herein.

<1> A method for manufacturing hydrogel particles, the method comprising a step of cooling an aqueous solution, in which a gel agent forming a non-crosslinked hydrogel is dissolved, down to a temperature lower than a gel point of the aqueous solution. In the step of cooling, a cooled substance of the aqueous solution is stirred at least at the gel point, and after the gel point has been reached, a stirring energy of 400 kW·s/m$^3$ or higher is applied to the cooled substance of the aqueous solution with stirring at a required stirring power per unit volume of 0.8 kW/m$^3$ or higher.

<2> The method of <1>, wherein stirring the aqueous solution or the cooled substance thereof is continuously performed over a period from before the temperature of the aqueous solution or the cooled substance thereof reaches the gel point to after the gel point has been reached.

<3> The method of <1> or <2>, wherein the stirring is performed while the temperature of the cooled substance of the aqueous solution is maintained at a certain temperature or within a certain temperature range equal to or lower than the gel point.

<4> The method of any one of <1> to <3>, wherein a temperature difference between the temperature of the cooled substance of the aqueous solution during the stirring and the gel point is suitably 0°C or larger, more suitably 1.0°C or larger, and still more suitably 1.5°C or larger, and is also suitably smaller than 20.0°C, more suitably 18.0°C or smaller, still more suitably 12.0°C or smaller, yet more suitably 7.0°C or smaller, and even still more suitably 3.0°C or smaller.

<5> The method of any one of <1> to <3>, wherein a temperature difference between the temperature of the cooled substance of the aqueous solution during the stirring and the gel point is 0°C or larger.

<6> The method of any one of <1> to <3>, wherein a temperature difference between the temperature of the cooled substance of the aqueous solution during the stirring and the gel point is 1.0°C or larger.

<7> The method of any one of <1> to <3>, wherein a temperature difference between the temperature of the cooled substance of the aqueous solution during the stirring and the gel point is 1.5°C or larger.

<8> The method of any one of <1> to <7>, wherein a temperature difference between the temperature of the cooled substance of the aqueous solution during the stirring and the gel point is 20.0°C or smaller.

<9> The method of any one of <1> to <7>, wherein a temperature difference between the temperature of the cooled substance of the aqueous solution during the stirring and the gel point is 18.0°C or smaller.

<10> The method of any one of <1> to <7>, wherein a temperature difference between the temperature of the cooled substance of the aqueous solution during the stirring and the gel point is 12.0°C or smaller.

<11> The method of any one of <1> to <7>, wherein a temperature difference between the temperature of the cooled substance of the aqueous solution during the stirring and the gel point is 7.0°C or smaller.

<12> The method of any one of <1> to <7>, wherein a temperature difference between the temperature of the cooled substance of the aqueous solution during the stirring and the gel point is 3.0°C or smaller.

<13> The method of any one of <1> to <12>, wherein the required stirring power per unit volume is suitably 1.0 kW/m$^3$ or higher, more suitably 1.1 kW/m$^3$ or higher, and still more suitably 1.2 kW/m$^3$ or higher, and is also suitably 15 kW/m$^3$ or lower, more suitably 10 kW/m$^3$ or lower, and still more suitably 7.0 kW/m$^3$ or lower.

<14> The method of any one of <1> to <13>, wherein the stirring energy is suitably 600 kW·s/m$^3$ or higher, more suitably 800 kW·s/m$^3$ or higher, still more suitably 1000 kW·s/m$^3$ or higher, yet more suitably 1500 kW·s/m$^3$ or higher, and even still more suitably 2000 kW·s/m$^3$ or higher, and is also suitably 15000 kW·s/m$^3$ or lower, more suitably 12000 kW·s/m$^3$ or lower, still more suitably 10000 kW·s/m$^3$ or lower, yet more suitably 8000 kW·s/m$^3$ or lower, even still more suitably 6000 kW·s/m$^3$ or lower, and most suitably 4000 kW·s/m$^3$ or lower.

<15> The method of any one of <1> to <14>, wherein the stirring energy is suitably 4000 kW·s/m$^3$ or higher, more suitably 5000 kW·s/m$^3$ or higher, still more suitably 6000 kW·s/m$^3$ or higher, and yet more suitably 7000 kW·s/m$^3$ or higher, and is also suitably 13000 kW·s/m$^3$ or lower, more suitably 11000 kW·s/m$^3$ or lower, still more suitably 10000 kW·s/m$^3$ or lower, and yet more suitably 9000 kW·s/m$^3$ or lower.

<16> The method of any one of <1> to <15>, wherein the stirring is changed to stirring at a required stirring power per unit volume lower than 0.8 kW/m$^3$ to be completed or is stopped to be completed.

<17> The method of <16>, wherein after the stirring is completed, the cooled substance of the aqueous solution is stirred at a required stirring power per unit volume lower than 0.8 kW/m$^3$ for aging or is left to stand still for aging.

<18> The method of <17>, wherein the aging is performed while the temperature of the cooled substance of the aqueous solution is being maintained at a certain temperature or within a certain temperature range equal to or lower than the gel point.

<19> The method of <17> or <18>, wherein a temperature difference between an aging temperature for the aging and the gel point is suitably 0°C or larger, more suitably 1.0°C or larger, and still more suitably 1.5°C or larger, and is also suitably smaller than 20.0°C, more suitably 18.0°C or smaller, still more suitably 12.0°C or smaller, yet more suitably 7.0°C or smaller, and even still more suitably 3.0°C or smaller.

<20> The method of any one of <17> to <19>, wherein the temperature difference between the aging temperature of the aging and the temperature of the cooled substance of the aqueous solution during the stirring is suitably 10.0°C or smaller, more suitably 5.0°C or smaller, and still more suitably 3.0°C or smaller.

<21> The method of any one of <17> to <20>, wherein an aging temperature of the aging is the same as the temperature of the cooled substance of the aqueous solution when the stirring has just been completed.

<22> The method of any one of <17> to <21>, wherein an aging time for the aging is suitably 10 minutes or longer, more suitably 30 minutes or longer, and still more suitably 60 minutes or longer, and is also suitably 300 minutes or shorter, more suitably 180 minutes or shorter, and still more suitably 120 minutes or shorter.

<23> The method of any one of <1> to <22>, wherein a temperature of the aqueous solution before being cooled in the step of cooling is equal to or higher than a dissolution temperature of the gel agent and also equal to or lower than a boiling point of water.

<24> The method of any one of <1> to <23>, wherein an average cooling speed in the step of cooling is suitably 0.1°C/min or higher, more suitably 0.5°C/min or higher, and still more suitably 0.8°C/min or higher, and is also suitably 5°C/min or lower, more suitably 4°C/min or lower, and still more suitably 3°C/min or lower.

<25> The method of any one of <1> to <24>, wherein a temperature of the hydrogel particles obtained in the step of cooling is suitably equal to or lower than the gel point, more suitably a temperature lower than the gel point by 1.0°C or more, and still more suitably a temperature lower than the gel point by 1.5°C or more.

<26> The method of any one of <1> to <25>, wherein a volume-mean particle size of the hydrogel particles obtained in the step of cooling is suitably 0.100 $\mu$m or larger, more suitably 10.0 $\mu$m or larger, and still more suitably 20.0 $\mu$m or larger, and is also suitably 10000 $\mu$m or smaller, more suitably 1000 $\mu$m or smaller, and still more suitably 600 $\mu$m or smaller.

<27> The method of any one of <1> to <26>, wherein the yield of the hydrogel particles obtained in the step of cooling is suitably 70 mass% or higher, more suitably 80 mass% or higher, and still more suitably 90 mass% or higher.

<28> The method of any one of <1> to <27>, wherein the gel agent contains one, two, or more selected from the group consisting of agar, carrageenan, gellan gum, xanthan gum, and high methoxyl pectin.

<29> The method of <28>, wherein the gel agent is agar.

<30> The method of <29>, wherein the temperature of the aqueous solution before being cooled in the step of cooling is suitably 75°C or higher and more suitably 80°C or higher, and is also suitably 100°C or lower.

<31> The method of <29> or <30>, wherein the temperature of the aqueous solution before being cooled in the step of cooling is suitably in the range of 75°C to 100°C and more suitably in the range of 80°C to 100°C.

<32> The method of any one of <29> to <31>, wherein a temperature of the hydrogel particles obtained in the step of cooling is suitably 5.0°C or higher, more suitably 8.0°C or higher, and still more suitably 10.0°C or higher, and is also suitably 35.5°C or lower, more suitably 34.5°C or lower, and still more suitably 34.0°C or lower.

<33> The method of any one of <1> to <32>, wherein a content of the gel agent in the aqueous solution is suitably 0.10 mass% or higher, more suitably 0.30 mass% or higher, still more suitably 0.40 mass% or higher, and yet more suitably 0.50 mass% or higher, and is also suitably 8.0 mass% or lower, more suitably 7.0 mass% or lower, still more suitably 6.0 mass% or lower, yet more suitably 5.0 mass% or lower, and even still more suitably 3.0 mass% or lower.

<34> The method of any one of <1> to <33>, wherein the gel point of the aqueous solution is suitably 30°C or higher, and is also suitably 50°C or lower and more suitably 45°C or lower.

<35> The method of any one of <1> to <34>, wherein the aqueous solution is a dispersion in which dispersed particles are distributed.

<36> The method of <35>, wherein the dispersed particles are one, two, or more selected from the group consisting of oil components, water-insoluble complexes containing catechins, and powders for cosmetic products.

<37> The method of <35> or <36>, wherein the content of the dispersed particles in the aqueous solution is suitably 1 mass% or higher, more suitably 2 mass% or higher, and still more suitably 3 mass% or higher, and is also suitably 60 mass% or lower, more suitably 55 mass% or lower, and still more suitably 50 mass% or lower.

<38> The method of any one of <35> to <37>, wherein the content of the dispersed particles in the aqueous solution is suitably in the range of 1 mass% to 60 mass%, more suitably in the range of 2 mass% to 55 mass%, and still more suitably in the range of 3 mass% to 50 mass%.

<39> The method of any one of <35> to <38>, wherein the aqueous solution contains at least one of a dispersant or an emulsifier.

<40> The method of <39>, wherein the at least one of a dispersant or an emulsifier is one, two, or more selected from the group consisting of polymer emulsifying and dispersing agents, anionic surfactants, cationic surfactants, nonionic surfactants, and amphoteric surfactants.

<41> The method of <39> or <40>, wherein the content of the at least one of a dispersant or an emulsifier in the aqueous solution is suitably 0.01 mass% or higher, more suitably 0.05 mass% or higher, and still more suitably 0.1 mass% or higher, and is also suitably 5 mass% or lower, more suitably 3 mass% or lower, and still more suitably 1 mass% or lower.

<42> The method of any one of <1> to <41>, wherein in the step of cooling, the aqueous solution and the cooled substance of the aqueous solution are cooled in a single stirring tank, and also the cooled substance of the aqueous solution is stirred therein.

<43> A method for manufacturing a cosmetic product, wherein the hydrogel particles obtained through the method of any one of <1> to <42> are blended with a cosmetic component into a liquid phase.

<44> The method of <43>, wherein the obtained hydrogel particles and the cosmetic component are put into a stirring tank that is different from a stirring tank in which the hydrogel particles have been manufactured.

<45> A method for manufacturing a cosmetic product, wherein the hydrogel particles obtained by the method of any one of <1> to <42> are put into a liquid phase, and are also crushed by a crushing method.

<46> The method of <45>, wherein the crushing of the hydrogel particles is performed by passing the liquid phase containing the hydrogel particles through a sieve.

<47> The method of <46>, wherein the liquid phase containing the hydrogel particles is passed through the sieve a plurality of times.

<48> The method of <47>, wherein the liquid phase containing the hydrogel particles is passed through a plurality of sieves that are arranged in series.

<49> The method of <48>, wherein the number of the sieves is suitably two or greater and more suitably three or greater, and is also suitably ten or less and more suitably five or less.

<50> The method of <48> or <49>, wherein the number of the sieves is suitably in the range of two to ten, and more suitably in the range of three to five.

<51> The method of any one of <48> to <50>, wherein the sieves include sieves having mutually different apertures.

<52> The method of <51>, wherein the sieves are arranged in the descending order of their apertures such that one of the sieves having the largest aperture is arranged most upstream of any other one of the sieves.

<53> The method of <52>, wherein in each adjacent pair of sieves having mutually different apertures among the sieves arranged in the descending order of their apertures such that the sieve having the largest aperture is arranged most upstream, the ratio of the aperture of the downstream sieve of the pair to the aperture of the upstream sieve thereof is suitably 1% or higher, more suitably 10% or higher, and still more suitably 20% or higher, and is also suitably 100% or lower, more suitably 90% or lower, and still more suitably 80% or lower.

<54> The method of any one of <52> to <53>, wherein among the sieves arranged in the descending order of their apertures such that the sieve having the largest aperture is arranged most upstream, the ratio of the aperture of the most downstream sieve to the aperture of the most upstream sieve is suitably 0.1% or higher, more suitably 1% or higher, and still more suitably 5% or higher, and is also suitably 100% or lower, more suitably 90% or lower, and still more suitably 80% or lower.

<55> The method of any one of <46> to <54>, wherein the aperture of each sieve is suitably 1 $\mu$m or larger, more suitably 10 $\mu$m or larger, and still more suitably 50 $\mu$m or larger, and is also suitably 1000 $\mu$m or smaller, more suitably 800 $\mu$m or smaller, still more suitably 600 $\mu$m or smaller, yet more suitably 500 $\mu$m or smaller, and even still more suitably 400 $\mu$m or smaller.

<56> The method of any one of <45> to <55>, wherein into the liquid phase, a cosmetic component is put in advance together with the hydrogel particles before the crushing.

<57> The method of any one of <43> to <56>, wherein a liquid that forms the liquid phase is one, two, or more selected from the group consisting of water, liquid oils, and organic solvents.

<58> The method of <57>, wherein the liquid that forms the liquid phase is water, an aqueous solution, or a water dispersion.

<59> The method of <43>, <44>, or <56>, wherein the cosmetic component is one, two, or more selected from the group consisting of polyether-modified silicones, cosmetic oils, humectants, intercellular lipids, ultraviolet absorbents, vitamins, chelating agents, pH adjusting agents, antiseptic agents, dyes, perfumes, medical components, emulsifying agents, cleaning agents, and antioxidants.

<60> Use of the hydrogel particles obtained by the method of any one of <1> to <44>, in the method of any one of <43> to <59>.

Examples

Production tests with hydrogel particles of Examples 1 to 18 and Comparative Examples 1 to 3 were conducted. Configurations of these examples are illustrated also in Tables 1 to 7.

(Example 1)

Ion-exchanged water was put in a blending tank, and agar (UP-16 manufactured by Ina Food Industry Co.,

Ltd., jelly strength: $600 \pm 30$ g/cm$^2$) as a gel agent and silica (SUNSPHERE H-121 manufactured by AGC Si-Tech Co., Ltd.) were put therein. This mixture was heated at 90.0°C to prepare 2300 g of gel-agent aqueous solution in which the agar was dissolved. The gel point was 35.5°C. The composition of the mixture was set such that the content of agar was 1.2 mass%, the content of silica was 6.7 mass%, and the remainder was the ion-exchanged water.

With a disperser (T. K. Robomix manufactured by PRIMIX Corporation), the gel-agent aqueous solution in the blending tank was cooled from 90.0°C to 33.3°C while being stirred by rotating the impeller at a rotational speed of 3900 rpm. In other words, the gel-agent aqueous solution or the cooled substance thereof was stirred continuously over a period from before the temperature of the gel-agent aqueous solution or the cooled substance thereof reached the gel point to after the gel point had been reached. At this time, the required stirring power per unit volume was 6.6 kW/m$^3$.

While the temperature of the cooled substance of the gel-agent aqueous solution was being maintained at 33.3°C, 10 minutes later after the gel point had been reached, that is, when the stirring energy applied to the cooled substance of the gel-agent aqueous solution by the stirring had reached 4000 kW·s/m$^3$ after the gel point had been reached, the cooled substance of the gel-agent aqueous solution obtained in the blending tank was sampled as Example 1-1. The volume-mean particle size of hydrogel particles of the cooled substance of the gel-agent aqueous solution thus sampled as Example 1-1 was measured by a laser diffraction/scattering method with a laser diffraction /scattering particle size distribution analyzer (LA-960S manufactured by HORIBA, Ltd.), and was determined to be 488 $\mu$m. For Example 1-1, the average cooling speed during a period from the cooling start time to the time when the stirring had been completed for the sampling was 2.7°C/min.

20 minutes later after the gel point had been reached, that is, when the stirring energy applied to the cooled substance of the gel-agent aqueous solution by the stirring had reached 8000 kW·s/m$^3$ after the gel point had been reached, the cooled substance of the gel-agent aqueous solution obtained in the blending tank was sampled as Example 1-2. The volume-mean particle size of the hydrogel particles thereof was measured in the same manner as in Example 1-1, and was determined to be 264 $\mu$m. The average cooling speed for Example 1-2 was 1.8°C/min.

30 minutes later after the gel point had been reached, that is, when the stirring energy applied to the cooled substance of the gel-agent aqueous solution by the stirring had reached 12000 kW·s/m$^3$ after the gel point had been reached, the cooled substance of the gel-agent aqueous solution obtained in the blending tank was sampled as Example 1-3. The volume-mean particle size of the hydrogel particles thereof was measured in the same manner as in Example 1-1, and was determined to be 77.0 $\mu$m. The average cooling speed for Example 1-3 was 1.4°C/min.

The temperature was 33.3°C at sampling of each of Examples 1-1 to 1-3, that is, at the stirring completion time thereof. Thus, after the gel point had been reached, the temperature of the cooled substance of the gel-agent aqueous solution during the stirring was 33.3 to 35.5°C, and accordingly the temperature difference between this temperature and the gel point was 0 to 2.2°C.

(Examples 2 to 5 and Comparative Examples 1 and 2)

Operation that is the same as in Example 1-1 was performed except that the stirring was stopped 1.13 minutes later after the gel point had been reached, that is, when the stirring energy applied to the cooled substance of the gel-agent aqueous solution by the stirring had reached 450 kW·s/m$^3$ after the gel point had been reached. Hydrogel particles of the cooled substance of the gel-agent aqueous solution obtained in the blending tank were used as Example 2. The volume-mean particle size of the hydrogel particles of Example 2 was measured to be 660 $\mu$m. The average cooling speed for Example 2 was 3.0°C/min. The yield of the hydrogel particles of Example 2 was 20 mass%. The yield of the hydrogel particles was determined as a percentage, to the mass of the prepared gel-agent aqueous solution, of the mass of hydrogel particles that were collected by passing the cooled substance of the gel-agent aqueous solution in the blending tank through a sieve ($\phi$200: manufactured by Tokyo Screen Co., Ltd., having an aperture of 5.6 mm and a wire diameter of 1.6 mm) (the same applies to the following).

Operation that is the same as in Example 2 was performed except that the stirring was stopped 2.0 minutes later after the gel point had been reached, that is, when the stirring energy applied to the cooled substance of the gel-agent aqueous solution by the stirring had reached 800 kW·s/m$^3$ after the gel point had been reached. Hydrogel particles of the cooled substance of the gel-agent aqueous solution obtained in the blending tank were used as Example 3. The volume-mean particle size of the hydrogel particles of Example 3 was measured to be 510 $\mu$m. The average cooling speed for Example 3 was 3.2°C/min. The yield of the hydrogel particles of Example 3 was 50 mass%.

Operation that is the same as in Example 2 was performed except that the stirring 2.5 was stopped minutes later after the gel point had been reached, that is, when the stirring energy applied to the cooled substance of the gel-agent aqueous solution by the stirring had reached 1000 W·s/m$^3$ after the gel point had been reached. Hydrogel particles of the cooled substance of the gel-agent aqueous solution obtained in the blending tank were used as Example 4. The volume-mean particle size of the hydrogel particles of Example 4 was measured to be 490 $\mu$m. The average cooling speed for Example 4 was 3.0°C/min. The yield of the hydrogel particles of Example 4 was 99 mass%.

Operation that is the same as in Example 2 was performed except that the stirring was stopped 4.3 minutes later after the gel point had been reached, that is, when the stirring energy applied to the cooled substance of the gel-agent aqueous solution by the stirring had reached 1700 kW·s/m$^3$ after the gel point had been reached. Hydrogel particles of the cooled substance of the gel-agent aqueous solution obtained in the blending tank were used as Example 5. The volume-mean particle size of the hydrogel particles of Example 5 was measured to be 480 $\mu$m. The average cooling speed for Example 5 was 3.0°C/min. The yield of the hydrogel particles of Example 5 was 99 mass%.

Operation that is the same as in Example 2 was performed except that the stirring was stopped 0.38 minute later after the gel point had been reached, that is, when the stirring energy applied to the cooled substance of the gel-agent aqueous solution by the stirring had reached 150 kW·s/m$^3$ after the gel point had been reached. The cooled substance of the gel-agent aqueous solution obtained in the blending tank was used as Comparative Example 1. Comparative Example 1 could not be obtained in the form of hydrogel particles. The average cooling speed for Comparative Example 1 was 2.7°C/min.

Operation that is the same as in Example 11 was performed except that the stirring was stopped 0.76 minute later after the gel point had been reached, that is, when the stirring energy applied to the cooled substance of the gel-agent aqueous solution by the stirring had reached 300 kW·s/m$^3$ after the gel point had been reached. The cooled substance of the gel-agent aqueous solution obtained in the blending tank was used as Comparative Example 2. Comparative Example 2 could not be obtained in the form of hydrogel particles. The average cooling speed for Comparative Example 2 was 2.7°C/min.

(Examples 6 to 8)

Operation that is the same as in Example 1-1 was performed except that the gel-agent aqueous solution was cooled to 30.2°C and the stirring was stopped when the stirring energy applied to the cooled substance of the gel-agent aqueous solution by the stirring had reached 4000 kW·s/m$^3$ after the gel point had been reached. Hydrogel particles of the cooled substance of the gel-agent aqueous solution obtained in the blending tank were used as Example 6. The volume-mean particle size of the hydrogel particles of Example 6 was measured to be 37.0 $\mu$m. The average cooling speed for Example 6 was 2.4°C/min. The yield of the hydrogel particles of Example 6 was 70 mass%.

Operation that is the same as in Example 6 was performed except that the stirring was stopped when the stirring energy applied to the cooled substance of the gel-agent aqueous solution by the stirring had reached 8000 kW·s/m$^3$ after the gel point had been reached. Hydrogel particles of the cooled substance of the gel-agent aqueous solution obtained in the blending tank were used as Example 7. The volume-mean particle size of the hydrogel particles of Example 7 was measured to be 19.0 $\mu$m. The average cooling speed for Example 7 was 1.7°C/min. The yield of the hydrogel particles of Example 7 was 70 mass%.

Operation that is the same as in Example 6 was performed except that the stirring was stopped when the stirring energy applied to the cooled substance of the gel-agent aqueous solution by the stirring had reached 12000 kW·s/m$^3$ after the gel point had been reached. Hydrogel particles of the cooled substance of the gel-agent aqueous solution obtained in the blending tank were used as Example 8. The volume-mean particle size of the hydrogel particles of Example 8 was measured to be 19.0 $\mu$m. The average cooling speed for Example 8 was 1.3°C/min. The yield of the hydrogel particles of Example 8 was 70 mass%.

Because the temperature at the stirring completion time in each of Examples 6 to 8 was 30.2°C, the temperature of the cooled substance of the gel-agent aqueous solution during the stirring was 30.2 to 35.5°C after the gel point had been reached. Thus, the temperature difference between this temperature and the gel point was 0 to 5.3°C.

(Examples 9 to 11)

Operation that is the same as in Example 6 was performed except that the gel-agent aqueous solution was cooled to 15.0°C. Hydrogel particles of the cooled substance of the gel-agent aqueous solution obtained in the blending tank were used as Example 9. The volume-mean particle size of the hydrogel particles of Example 9 was measured to be 35.0 $\mu$m. The average cooling speed for Example 9 was 3.0°C/min. The yield of the hydrogel particles of Example 9 was 10 mass%.

Operation that is the same as in Example 9 was performed except that the stirring was stopped when the stirring energy applied to the cooled substance of the gel-agent aqueous solution by the stirring had reached 8000 kW·s/m$^3$ after the gel point had been reached. Hydrogel particles of the cooled substance of the gel-agent aqueous solution obtained in the blending tank were used as Example 10. The volume-mean particle size of the hydrogel particles of Example 10 was measured to be 30.0 $\mu$m. The average cooling speed for Example 10 was 2.0°C/min. The yield of the hydrogel particles of Example 10 was 10 mass%.

Operation that is the same as in Example 9 was performed except that the stirring was stopped when the stirring energy applied to the cooled substance of the gel-agent aqueous solution by the stirring had reached 12000 kW·s/m$^3$ after the gel point had been reached. Hydrogel particles of the cooled substance of the gel-agent aqueous solution obtained in the blending tank were used as Example 11. The volume-mean particle size of the hydrogel particles of Example 11 was measured to be 25.0 μm. The average cooling speed for Example 11 was 1.5°C/min. The yield of the hydrogel particles of Example 11 was 10 mass%.

Because the temperature at the stirring completion time in each of Examples 9 to 11 was 15.0°C, the temperature of the cooled substance of the gel-agent aqueous solution during the stirring was 15.0 to 35.5°C after the gel point had been reached. Thus, the temperature difference between this temperature and the gel point was 0 to 20.5°C.

[Table 1]

| | Example | | |
|---|---|---|---|
| | 1-1 | 1-2 | 1-3 |
| Required Stirring Power (kW/m$^3$) | 6.6 | 6.6 | 6.6 |
| Stirring Time below Gel Point (min) | 10 | 20 | 30 |
| Stirring Energy (kW·s/m$^3$) | 4000 | 8000 | 12000 |
| Volume-Mean Particle Size of Hydrogel Particles (μm) | 488 | 264 | 77.0 |
| Average Cooling Speed (°C/min) | 2.7 | 1.8 | 1.4 |
| Temperature Range during Stirring (°C) | 35.5 \| 33.3 | 35.5 \| 33.3 | 35.5 \| 33.3 |
| Temperature Difference between Temperature during Stirring and Gel Point (°C) | 0 \| 2.2 | 0 \| 2.2 | 0 \| 2.2 |
| Temperature at Stirring Completion Time (°C) | 33.3 | 33.3 | 33.3 |
| Temperature at Particle-Size Measurement Time (°C) | 33.3 | 33.3 | 33.3 |

[Table 2]

| | Example | | | | | | | Comparative Example | |
|---|---|---|---|---|---|---|---|---|---|
| | 2 | 3 | 4 | 5 | 1-1 | 1-2 | 1-3 | 1 | 2 |
| Required Stirring Power (kW/m$^3$) | 6.6 | 6.6 | 6.6 | 6.6 | 6.6 | 6.6 | 6.6 | 6.6 | 6.6 |
| Stirring Time below Gel Point (min) | 1.13 | 2.0 | 2.5 | 4.3 | 10 | 20 | 30 | 0.38 | 0.76 |
| Stirring Energy (kW·s/m$^3$) | 450 | 800 | 1000 | 1700 | 4000 | 8000 | 12000 | 150 | 300 |
| Volume-Mean Particle Size of Hydrogel Particles (μm) | 660 | 510 | 490 | 480 | 488 | 264 | 77.0 | - | - |
| Average Cooling Speed (°C/min) | 3.0 | 3.2 | 3.0 | 3.0 | 2.7 | 1.8 | 1.4 | 2.7 | 2.7 |
| Temperature Range during Stirring (°C) | 35.5 \| 33.3 | 35.5 \| 33.3 | 35.5 \| 33.3 | 35.5 \| 33.3 | 35.5 \| 33.3 | 35.5 \| 33.3 | 35.5 \| 33.3 | 35.5 \| 33.3 | 35.5 \| 33.3 |
| Temperature Difference between Temperature during Stirring and Gel Point (°C) | 0 \| 2.2 | 0 \| 2.2 | 0 \| 2.2 | 0 \| 2.2 | 0 \| 2.2 | 0 \| 2.2 | 0 \| 2.2 | 0 \| 2.2 | 0 \| 2.2 |
| Temperature at Stirring Completion Time (°C) | 33.3 | 33.3 | 33.3 | 33.3 | 33.3 | 33.3 | 33.3 | 33.3 | 33.3 |
| Temperature at Particle-Size Measurement Time (°C) | 33.3 | 33.3 | 33.3 | 33.3 | 33.3 | 33.3 | 33.3 | - | - |

(continued)

| | Example | | | | | | | Comparative Example | |
|---|---|---|---|---|---|---|---|---|---|
| | 2 | 3 | 4 | 5 | 1-1 | 1-2 | 1-3 | 1 | 2 |
| Yield of Hydrogel Particles (mass%) | 20 | 50 | 99 | 99 | - | - | - | - | - |

[Table 3]

| | Example | | |
|---|---|---|---|
| | 6 | 7 | 8 |
| Required Stirring Power (kW/m$^3$) | 6.6 | 6.6 | 6.6 |
| Stirring Time below Gel Point (min) | 10 | 20 | 30 |
| Stirring Energy (kW·s/m$^3$) | 4000 | 8000 | 12000 |
| Volume-Mean Particle Size of Hydrogel Particles ($\mu$m) | 37.0 | 19.0 | 19.0 |
| Average Cooling Speed (°C/min) | 2.4 | 1.7 | 1.3 |
| Temperature Range during Stirring (°C) | 35.5 \| 30.2 | 35.5 \| 30.2 | 35.5 \| 30.2 |
| Temperature Difference between Temperature during Stirring and Gel Point (°C) | 0 \| 5.3 | 0 \| 5.3 | 0 \| 5.3 |
| Temperature at Stirring Completion Time (°C) | 30.2 | 30.2 | 30.2 |
| Temperature at Particle-Size Measurement Time (°C) | 30.2 | 30.2 | 30.2 |
| Yield of Hydrogel Particles (mass%) | 70 | 70 | 70 |

[Table 4]

| | Example | | |
|---|---|---|---|
| | 9 | 10 | 11 |
| Required Stirring Power (kW/m$^3$) | 6.6 | 6.6 | 6.6 |
| Stirring Time below Gel Point (min) | 10 | 20 | 30 |
| Stirring Energy (kW·s/m$^3$) | 4000 | 8000 | 12000 |
| Volume-Mean Particle Size of Hydrogel Particles ($\mu$m) | 35.0 | 30.0 | 25.0 |
| Average Cooling Speed (°C/min) | 3.0 | 2.0 | 1.5 |
| Temperature Range during Stirring (°C) | 35.5 \| 15.0 | 35.5 \| 15.0 | 35.5 \| 15.0 |
| Temperature Difference between Temperature during Stirring and Gel Point (°C) | 0 \| 20.5 | 0 \| 20.5 | 0 \| 20.5 |
| Temperature at Stirring Completion Time (°C) | 15.0 | 15.0 | 15.0 |
| Temperature at Particle-Size Measurement Time (°C) | 15.0 | 15.0 | 15.0 |

(continued)

|  | Example | | |
|---|---|---|---|
|  | 9 | 10 | 11 |
| Yield of Hydrogel Particles (mass%) | 10 | 10 | 10 |

According to Tables 1 to 4, it was observed that the particle size of the hydrogel particles tended to depend on the stirring energy, and also it was found that this tendency increased as the temperature difference between the temperature during the stirring and the gel point decreased. Furthermore, from Comparative Examples 1 and 2, it was found that the form of hydrogel particles could not be obtained when the stirring energy was low.

(Example 12)

Operation that is the same as in Example 6 was performed except that the gel-agent aqueous solution was cooled to 22.5°C. Hydrogel particles of the cooled substance of the gel-agent aqueous solution obtained in the blending tank were used as Example 12. The volume-mean particle size of the hydrogel particles of Example 12 was measured to be 35.0 $\mu$m. The average cooling speed for Example 12 was 2.7°C/min. The yield of the hydrogel particles of Example 12 was 40 mass%. Because the temperature at the stirring completion time in Example 12 was 22.5°C, the temperature of the cooled substance of the gel-agent aqueous solution during the stirring was 22.5 to 35.5°C after the gel point had been reached. Thus, the temperature difference between this temperature and the gel point was 0 to 13.0°C.

[Table 5]

|  | Example | | |
|---|---|---|---|
|  | 6 | 12 | 9 |
| Required Stirring Power (kW/m$^3$) | 6.6 | 6.6 | 6.6 |
| Stirring Time below Gel Point (min) | 10 | 10 | 10 |
| Stirring Energy (kW·s/m$^3$) | 4000 | 4000 | 4000 |
| Volume-Mean Particle Size of Hydrogel Particles ($\mu$m) | 37.0 | 35.0 | 35.0 |
| Average Cooling Speed (°C/min) | 2.4 | 2.7 | 3.0 |
| Temperature Range during Stirring (°C) | 35.5 \| 30.2 | 35.5 \| 22.5 | 35.5 \| 15.0 |
| Temperature Difference between Temperature during Stirring and Gel Point (°C) | 0 \| 5.3 | 0 \| 13.0 | 0 \| 20.5 |
| Temperature at Stirring Completion Time (°C) | 30.2 | 22.5 | 15.0 |
| Temperature at Particle-Size Measurement Time (°C) | 30.2 | 22.5 | 15.0 |
| Yield of Hydrogel Particles (mass%) | 70 | 40 | 10 |

According to Table 5, based on a comparison among Example 6, Example 12, and Example 9 in which the temperatures during the stirring were different from each other, it was found that the yield of hydrogel particles increased as the temperature difference between the temperature during the stirring and the gel point decreased. This can be seen in comparison between Example 7 and Example 10 and comparison between Example 8 and Example 11 in Tables 3 and 4.

(Examples 13 and 14)

Operation that is the same as in Example 1-1 was performed except that the stirring was stopped when the stirring energy applied to the cooled substance of the gel-agent aqueous solution by the stirring had reached 4000 kW·s/m$^3$ after the gel point had been reached, and also except that, after this stopping, the cooled substance of the gel-agent aqueous solution was cooled until the temperature thereof reached 15.0°C. Hydrogel particles of the cooled substance of the gel-agent aqueous solution obtained in the blending tank were used as Example 13. The volume-mean particle size of the hydrogel particles of Example 13 was measured to be 485 $\mu$m. The average cooling speed for Example 13 was 2.7°C/min. The yield of the hydrogel particles of Example 13 was 99 mass%. Because the temperature at the stirring completion time in Example 13 was 33.3°C, the temperature of the cooled substance of the gel-agent aqueous solution during the stirring was 33.3 to 35.5°C after the gel point had been reached. Thus, the temperature difference between this temperature and the gel point was 0 to 2.2°C.

Operation that is the same as in Example 13 was performed except that the gel-agent aqueous solution was cooled to 30.2°C. Hydrogel particles of the cooled substance of the gel-agent aqueous solution obtained in the blending tank were used as Example 14. The volume-mean particle size of the hydrogel particles of Example 14 was measured to be 37.0 $\mu$m. The average cooling speed for Example 14 was 2.4°C/min. The yield of the hydrogel particles of Example 14 was 70 mass%. Because the temperature at the stirring completion time in Example 14 was 30.2°C, the temperature of the cooled substance of the gel-agent aqueous solution during the stirring was 30.2 to 35.5°C after the gel point had been reached. Thus, the temperature difference between this temperature and the gel point was 0 to 5.3°C.

[Table 6]

| | Example | | | | |
|---|---|---|---|---|---|
| | 13 | 1-1 | 9 | 14 | 6 |
| Required Stirring Power (kW/m$^3$) | 6.6 | 6.6 | 6.6 | 6.6 | 6.6 |
| Stirring Time below Gel Point (min) | 10 | 10 | 10 | 10 | 10 |
| Stirring Energy (kW·s/m$^3$) | 4000 | 4000 | 4000 | 4000 | 4000 |
| Volume-Mean Particle Size of Hydrogel Particles ($\mu$m) | 485 | 488 | 35.0 | 37.0 | 37.0 |
| Average Cooling Speed (°C/min) | 2.7 | 2.7 | 3.0 | 2.4 | 2.4 |
| Temperature Range during Stirring (°C) | 35.5 \| 33.3 | 35.5 \| 33.3 | 35.5 \| 15.0 | 35.5 \| 30.2 | 35.5 \| 30.2 |
| Temperature Difference between Temperature during Stirring and Gel Point (°C) | 0 \| 2.2 | 0 \| 2.2 | 0 \| 20.5 | 0 \| 5.3 | 0 \| 5.3 |
| Temperature at Stirring Completion Time (°C) | 33.3 | 33.3 | 15.0 | 30.2 | 30.2 |
| Temperature at Particle-Size Measurement Time (°C) | 15.0 | 33.3 | 15.0 | 15.0 | 30.2 |
| Yield of Hydrogel Particles (mass%) | 99 | - | 10 | 70 | 70 |

According to Table 6, based on a comparison between Example 13 and Example 1-1 and a comparison between Example 14 and Example 6, it was found that the particle size of hydrogel particles did not substantially change even if cooling was performed after the stirring. By contrast, it was found that, in Example 9 in which cooling was performed to the same temperature as in Example 13 while the stirring was being continued, the particle size of the hydrogel particles was smaller than that in Example 13, and the yield of the hydrogel particles was also lower.

(Examples 15 to 18 and Comparative Example 3)

Operation that is the same as in Example 1-1 was performed except that the required stirring power per unit volume was 1.0 kW/m$^3$ and the stirring was stopped 67 minutes later after the gel point had been reached, that is, when the stirring energy applied to the cooled substance of the gel-agent aqueous solution by the stirring had reached 4000 kW·s/m$^3$ after the gel point had been reached. Hydrogel particles of the cooled substance of the gel-agent aqueous solution obtained in the blending tank were used as Example 15. The volume-mean particle size of the hydrogel particles of Example 15 was measured to be 30.0 $\mu$m. The average cooling speed for Example 15 was 0.7°C/min. The yield of the hydrogel particles of Example 15 was 30 mass%.

Operation that is the same as in Example 15 was performed except that the required stirring power per unit volume was 1.2 kW/m$^3$ and the stirring was stopped 56 minutes later after the gel point had been reached, that is, when the stirring energy applied to the cooled substance of the gel-agent aqueous solution by the stirring had reached 4000

kW·s/m$^3$ after the gel point had been reached. Hydrogel particles of the cooled substance of the gel-agent aqueous solution obtained in the blending tank were used as Example 16. The volume-mean particle size of the hydrogel particles of Example 16 was measured to be 35.0 $\mu$m. The average cooling speed for Example 16 was 0.8°C/min. The yield of the hydrogel particles of Example 16 was 66 mass%.

Operation that is the same as in Example 15 was performed except that the required stirring power per unit volume was 1.5 kW/m$^3$ and the stirring was stopped 44 minutes later after the gel point had been reached, that is, when the stirring energy applied to the cooled substance of the gel-agent aqueous solution by the stirring had reached 4000 kW·s/m$^3$ after the gel point had been reached. Hydrogel particles of the cooled substance of the gel-agent aqueous solution obtained in the blending tank were used as Example 17. The volume-mean particle size of the hydrogel particles of Example 17 was measured to be 45.0 $\mu$m. The average cooling speed for Example 17 was 1.0°C/min. The yield of the hydrogel particles of Example 17 was 80 mass%.

Operation that is the same as in Example 15 was performed except that the required stirring power per unit volume was 1.9 kW/m$^3$ and the stirring was stopped 35 minutes later after the gel point had been reached, that is, when the stirring energy applied to the cooled substance of the gel-agent aqueous solution by the stirring had reached 4000 kW·s/m$^3$ after the gel point had been reached. Hydrogel particles of the cooled substance of the gel-agent aqueous solution obtained in the blending tank were used as Example 18. The volume-mean particle size of the hydrogel particles of Example 18 was measured to be 65.0 $\mu$m. The average cooling speed for Example 18 was 1.1 °C/min. The yield of the hydrogel particles of Example 18 was 92 mass%.

Operation that is the same as in Example 15 was performed except that the required stirring power per unit volume was 0.5 kW/m$^3$ and the stirring was stopped 133 minutes later after the gel point had been reached, that is, when the stirring energy applied to the cooled substance of the gel-agent aqueous solution by the stirring had reached 4000 kW·s/m$^3$ after the gel point had been reached. The cooled substance of the gel-agent aqueous solution obtained in the blending tank was used as Comparative Example 3. Comparative Example 3 could not be obtained in the form of hydrogel particles. The average cooling speed for Comparative Example 3 was 0.4°C/min.

[Table 7]

|  | Example |  |  |  |  | Comparative Example |
|---|---|---|---|---|---|---|
|  | 15 | 16 | 17 | 18 | 1-1 | 3 |
| Required Stirring Power (kW/m$^3$) | 1.0 | 1.2 | 1.5 | 1.9 | 6.6 | 0.5 |
| Stirring Time below Gel Point (min) | 67 | 56 | 44 | 35 | 10 | 133 |
| Stirring Energy (kW·s/m$^3$) | 4000 | 4000 | 4000 | 4000 | 4000 | 4000 |
| Volume-Mean Particle Size of Hydrogel Particles ($\mu$m) | 30.0 | 35.0 | 45.0 | 65.0 | 488 | - |
| Average Cooling Speed (°C/min) | 0.7 | 0.8 | 1.0 | 1.1 | 2.7 | 0.4 |
| Temperature Range during Stirring (°C) | 35.5 \| 33.3 | 35.5 \| 33.3 | 35.5 \| 33.3 | 35.5 \| 33.3 | 35.5 \| 33.3 | 35.5 \| 33.3 |
| Temperature Difference between Temperature during Stirring and Gel Point (°C) | 0 \| 2.2 | 0 \| 2.2 | 0 \| 2.2 | 0 \| 2.2 | 0 \| 2.2 | 0 \| 2.2 |
| Temperature at Stirring Completion Time (°C) | 33.3 | 33.3 | 33.3 | 33.3 | 33.3 | 33.3 |
| Temperature at Particle-Size Measurement Time (°C) | 33.3 | 33.3 | 33.3 | 33.3 | 33.3 | - |
| Yield of Hydrogel Particles (mass%) | 30 | 66 | 80 | 92 | - | - |

According to Table 7, it was observed that the particle size of the hydrogel particles tended to depend on the required stirring power per unit volume. Furthermore, from Comparative Example 3, it was found that the form of hydrogel particles could not obtained when the required stirring power per unit volume was low.

INDUSTRIAL APPLICABILITY

The present invention is useful in the technical fields of a method for manufacturing hydrogel particles and a method for manufacturing a cosmetic product.

**Claims**

1. A method for manufacturing hydrogel particles, the method comprising a step of cooling an aqueous solution, in which a gel agent forming a non-crosslinked hydrogel is dissolved, down to a temperature lower than a gel point of the aqueous solution, wherein
in the step of cooling, a cooled substance of the aqueous solution is stirred at least at the gel point, and after the gel point has been reached, a stirring energy of 400 kW·s/m$^3$ or higher is applied to the cooled substance of the aqueous solution with stirring at a required stirring power per unit volume of 0.8 kW/m$^3$ or higher.

2. The method of claim 1, wherein
stirring the aqueous solution or the cooled substance of the aqueous solution is continuously performed over a period from before a temperature of the aqueous solution or a temperature of the cooled substance of the aqueous solution reaches the gel point to after the gel point has been reached.

3. The method of claim 1 or 2, wherein
an average cooling speed in the step of cooling is 0.1 °C/min or higher and 5°C/min or lower.

4. The method of any one of claims 1 to 3, wherein
a temperature difference between the temperature of the cooled substance of the aqueous solution during the stirring and the gel point is smaller than 20°C.

5. The method of any one of claims 1 to 4, wherein
the stirring is changed to stirring at a required stirring power per unit volume lower than 0.8 kW/m$^3$ to be completed or is stopped to be completed.

6. The method of claim 5, wherein
after the stirring is completed, the cooled substance of the aqueous solution is stirred at a required stirring power per unit volume lower than 0.8 kW/m$^3$ for aging or is left to stand still for aging.

7. The method of claim 6, wherein
an aging time for the aging is 10 minutes or longer.

8. The method of claim 6 or 7, wherein
a temperature difference between an aging temperature of the aging and the gel point is smaller than 20°C.

9. The method of any one of claims 6 to 8, wherein
a temperature difference between an aging temperature of the aging and the temperature of the cooled substance of the aqueous solution during the stirring is 10.0°C or smaller.

10. The method of any one of claims 1 to 9, wherein
a volume-mean particle size of the hydrogel particles is 0.100 μm or larger and 10000 μm or smaller.

11. A method for manufacturing a cosmetic product, wherein the hydrogel particles obtained through the method of any one of claims 1 to 10 are blended with a cosmetic component into a liquid phase.

<table>
<tr><td colspan="2" align="center">**INTERNATIONAL SEARCH REPORT**</td><td>International application No.</td></tr>
<tr><td colspan="2"></td><td>PCT/JP2017/041520</td></tr>
</table>

A.  CLASSIFICATION OF SUBJECT MATTER
Int.Cl. B01J2/08(2006.01)i, A61K8/02(2006.01)i, B01J13/00(2006.01)i

According to International Patent Classification (IPC) or to both national classification and IPC

B.  FIELDS SEARCHED

Minimum documentation searched (classification system followed by classification symbols)
Int.Cl. B01J2/08, A61K8/02, B01J13/00

Documentation searched other than minimum documentation to the extent that such documents are included in the fields searched

| | |
|---|---|
| Published examined utility model applications of Japan | 1922–1996 |
| Published unexamined utility model applications of Japan | 1971–2018 |
| Registered utility model specifications of Japan | 1996–2018 |
| Published registered utility model applications of Japan | 1994–2018 |

Electronic data base consulted during the international search (name of data base and, where practicable, search terms used)
JSTPlus/JMEDPlus/JST7580 (JDreamIII)

C.  DOCUMENTS CONSIDERED TO BE RELEVANT

| Category* | Citation of document, with indication, where appropriate, of the relevant passages | Relevant to claim No. |
|---|---|---|
| Y | JP 2005-082527 A (LION CORP.) 31 March 2005, claims, paragraphs [0036]-[0037], examples (Family: none) | 1-11 |
| Y | WO 2010/071095 A1 (KAO CORP.) 24 June 2010, paragraphs [0037]-[0049], table 2 & JP 2010-142711 A & JP 2010-275269 A & CN 102256694 A | 1-11 |
| P, A | WO 2016/194817 A1 (KAO CORP.) 08 December 2016 & JP 2016-221508 A | 1-11 |

☐  Further documents are listed in the continuation of Box C.　　☐  See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 06 February 2018 (06.02.2018) | 20 February 2018 (20.02.2018) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

# REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2007160277 A
- JP 2001097818 A
- JP 2003128588 A
- JP 2001342451 A
- JP 2010131479 A
- JP 2011136983 A
- JP H7100356 B
- JP H8252447 B
- JP H9141079 B
- JP H9141080 B
- JP H9141081 B
- JP H1053625 B
- JP 3329689 B
- JP H10330401 B
- JP H11106401 B
- JP 2007161683 A